# EUROPEAN PATENT APPLICATION

(11) **EP 1 707 171 A2**
(43) Date of publication of application: **04.10.2006**
(21) Application number: 06251744.6
(22) Date of filing: 30.03.2006
(51) Int. Cl.: A61F 13/82

(54) **Body attachable sanitary protection article including non-staining release member**

(30) Priority: 31.03.2005 US 95049
(71) Applicant: McNeil-PPC, Inc., Skillman, NJ 08558 (US)
(72) Inventor: Luzzi, Joseph M., Newtown, PA 18940 (US); Gannon, Elaine M., Hoboken, NJ 07030 (US); Johnson, Bruce C., Whiting, NJ 08759 (US); Moscherosch, H. Michael, Doylestown, PA 19801 (US); Rosenfeld, Leonard G., Yardley, PA 19067 (US)
(74) Representative: Mercer, Christopher Paul

(57) **Abstract**

An absorbent article including a liquid pervious cover, a liquid impermeable barrier, a body facing surface, an adhesive for securing the article to the body arranged on the body facing surface, and a removable release member for covering the adhesive prior to use, the release member being impervious to the migration of adhesive therethrough.

## Description

### FIELD OF THE INVENTION

The present invention relates to a body attachable sanitary protection article, such as a sanitary napkin, and in particular a body attachable sanitary protection article including a non-staining release member.

### BACKGROUND OF THE INVENTION

Various absorbent articles configured to be arranged adjacent the body to absorb body fluids such as menses, urine and the like are well known. With respect to feminine hygiene, napkins and liners have been developed for external use about the pudendal region of a female.

Securement of a sanitary napkin or liner during use is normally accomplished by attaching the sanitary garment by pressure sensitive adhesive to the wearer's undergarment. Napkins having wings or flaps that fold over the edges of the garment and are attached to the underside of the garment using an adhesive are also known.

The prior art also teaches sanitary protection articles that are intended to be secured directly to the body by an adhesive arranged on a body-facing surface of the article. For example, GB2284767A purports to disclose an absorbent article including an absorbent and an adhesive arranged adjacent the absorbent, the adhesive being designed to contact the wearer's body during use. U.S. Patent No. 6,213,993 purports to disclose a self-adhering absorbent article including a liquid permeable cover, an absorbent core, a liquid impermeable baffle, and a bodyside adhesive arranged on the cover for securing the article to the body.

Body attachable sanitary articles disclosed in the prior art are typically provided with a with a removable member, such as a release paper, that serves to protect the adhesive from inadvertently adhering to a surface prior to use. The release member often comprises a densified, bleached paper, formed from a fibrous slurry. The release paper may have a coating of silicone applied to a surface thereof to facilitate the easy removal of the paper prior to use.

A problem with body attachable sanitary articles of the type discussed above is that the adhesives used to secure the article body often stain the release paper covering the adhesive prior to use. This staining, which resembles "grease spots", creates an aesthetic concern for consumers since it suggests that the product is not hygienic. The staining of the release paper is caused by the migration of the plasticizing components in these types of adhesives being absorbed by the release paper. In addition to the aesthetic problems caused by the migration of the adhesive into the release paper, the migration of the adhesive may also compromise the body attachment performance of the product.

In view of the above there is a need for an improved body attachable sanitary article that overcomes the drawbacks and shortcomings of the articles disclosed in the

### prior art.

### SUMMARY OF THE INVENTION

According to one aspect of the invention, the present invention relates to an absorbent article including a liquid pervious cover, a liquid impermeable barrier; a body facing surface, an adhesive for securing the article to the body arranged on the body facing surface, a removable release member for covering the adhesive prior to use, the release member being impervious to the migration of any component of the adhesive therethrough.

According to another aspect of the invention, the present invention relates to an absorbent article including a liquid pervious cover, a liquid impermeable barrier, a body facing surface, an adhesive for securing the article to the body arranged on the body facing surface, wherein the adhesive is an adhesive gel based upon a styrene-ethylene-butylene-styrene (SEBS) block copolymer, a removable release member for covering the adhesive prior to use, the release member being impervious to the migration of any component of the adhesive therethrough.

According to yet another aspect of the invention, the present invention relates to a method for preventing a migration of an adhesive though a removable release member in a body attachable sanitary article, the method included the steps of providing a body facing surface, applying an adhesive to the body facing surface for securing the article to a user's body, applying a removable release member over the adhesive to protect the adhesive prior to use, providing said release member with a nonporous structure to prevent the migration of any component of the adhesive through the release member.

### BRIEF DESCRIPTION OF THE DRAWINGS

Examples of embodiments of the present invention will now be described with reference to the drawings, in which:
Fig. 1 is a perspective view a body attachable sanitary napkin in accordance with the prior art;
Fig. 2 is a perspective view of body attachable sanitary napkin according to the present invention, the release member thereof being partially broken away to show the cover layer;
Fig. 3 is an exploded perspective view of the sanitary napkin shown in Fig. 2;
Fig. 4a is a sectional view of the release member used in the sanitary napkin according to the present invention;
Fig. 4b is a sectional view of an alternate release member used in the sanitary napkin according to the present invention;
Fig. 5 is a perspective view of a body attachable sanitary napkin according to another embodiment of the present invention, the release member thereof being partially broken away to show the cover layer;
Fig. 6 is an exploded perspective view of the sanitary napkin shown in Fig. 5; and
Fig. 6a is a detailed perspective view of a portion of the sanitary napkin shown in Figs. 5 and 6.

### DETAILED DESCRIPTION OF THE INVENTION

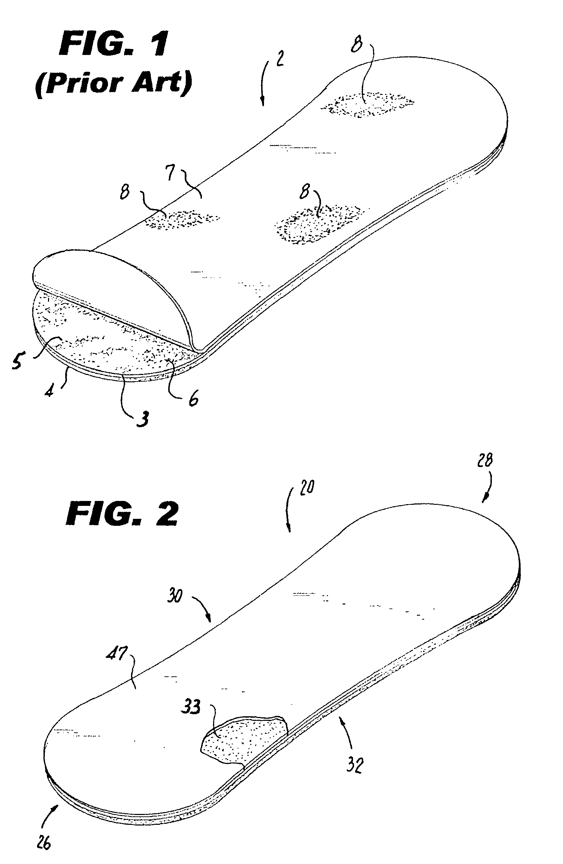

Referring to Fig. 1, there is shown a body attachable sanitary napkin 1 in accordance with the prior art. As shown, the sanitary napkin 1 includes a main body 2 including a cover 3 and a liquid impermeable barrier 4. Applied to the body facing surface 5 of the cover 3 is an adhesive 6 for securing the napkin to the body of a user. The napkin 1 further includes a release member 7 for covering the adhesive 6 prior to use. The release member 7 comprises a densified, bleached kraft paper, formed from a fibrous slurry, the paper having a coating of silicone applied thereto.

As shown in Fig. 1, a portion of the adhesive 6 has migrated through the release paper 7 thereby forming a stain 8. The stain 8 creates an aesthetic concern for consumers since it suggests that the product is not hygienic. It has been found that this staining problem is particularly troublesome when the adhesive 6 is an adhesive gel based upon a styrene-ethylene-butylene-styrene (SEBS) block copolymer.

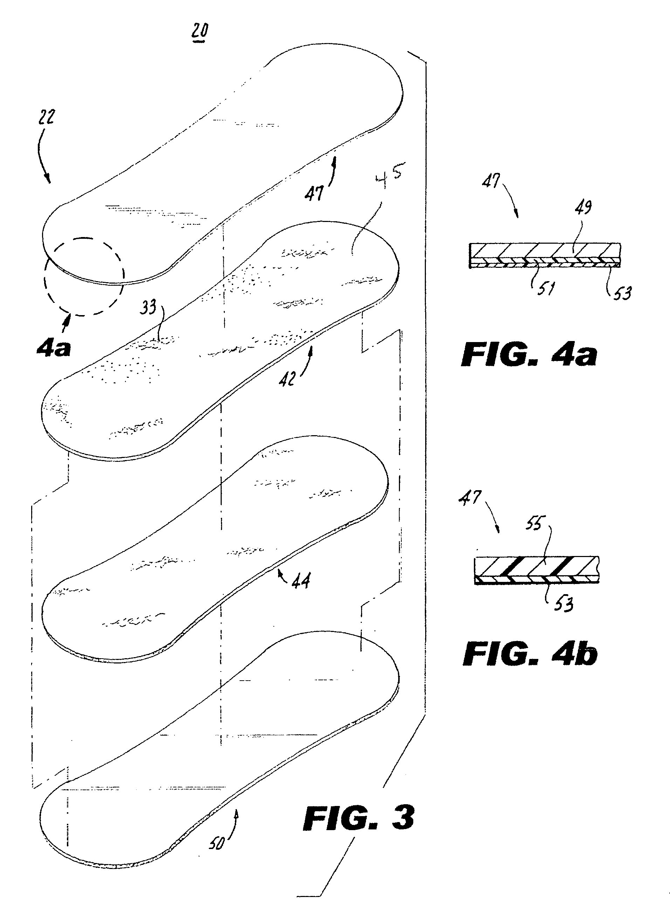

Referring to FIGS. 2 and 3, there is shown an embodiment of the present invention, a feminine sanitary napkin 20. The sanitary napkin 20 has a main body 22 with a first transverse side 26 defining a front portion thereof and a second transverse side 28 defining a rear portion thereof. The main body also has two longitudinal sides, namely a longitudinal side 30 and a longitudinal side 32.

As depicted in FIG. 3, the main body 22 is of a laminate construction and preferably comprises a fluid-permeable cover layer 42, an absorbent system 44 and a fluid-impervious barrier layer 50. The absorbent system 44 may comprise a single layer of material or may comprise multiple layers. For example, the absorbent system 44 may comprise a single layer core or it may include a transfer layer and a core.

In the embodiment of the invention shown in Figures 2 and 3, an adhesive 33 for securing the napkin 20 to the body of a user is applied to the body facing surface 45 of the cover 42.

The adhesive 33 employed in the article according to the present invention preferably has more than about 50% by weight of a liquid plasticizer, preferably more than about 65% by weight of a liquid plasticizer, and most preferably more than about 80% by weight of a liquid plasticizer. Suitable liquid plasticizers may include white oils, mineral oils, paraffinic process oils, polyethylene glycol, glycerin, polypropylene glycol, napthenic oils, and liquid polyterpenes. The liquid plasticizer preferably has a molecular weight of less than 1000 g/mole, more preferably less than 750 g/mole and most preferably less than 500 g/mole.

The adhesive 33 used in the article according to the present invention is preferably an adhesive based upon block copolymers, preferably, those which may include linear or radial co-polymer structures having the formula (A-B)ₓ wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or most preferably hydrogenated elastomers such as ethylene-butylene or ethylene-propylene or polyisobutylene, or combinations thereof, specifically, adhesives consisting of styrene-ethylene-butylene-styrene (SEBS) block copolymer and mineral oils, paraffinic or napthenic process oils, and optionally a suitable tackifying resins include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic/aromatic petroleum resins and the hydrogenated derivatives thereof, and combinations thereof.

The adhesive 33 may be of the type described in US Patent No. 6,191,189 to Cinelli et al. In particular, the adhesive may comprise:
from 0.5 to 20%, preferably 5% to 15%, by weight of a macromolecular polymeric substance or a mixture of such substances soluble or swellable in the below mentioned plasticiser(s). As not limiting examples such macromolecular or polymeric substances can be natural and/or synthetic such as natural gums or derivatives such as natural gums and gelatins, their derivatives and alginates; polyacrylics; polyvinyl alcohol; polyethylene oxide; polyvinylpyrrolidone (PVP) or polyvinylethers, their copolymers and derivatives; cellulose derivatives; Block Copolymer Thermoplastic Elastomers and preferably Styrenic Block Copolymers and more preferably the hydrogenated grades Styrol/Ethylene-Butylene/Styrol (SEBS), Styrene/Isoprene/Styrene (SIS), and Styrol/Ethylene-Propylene/Styrol (SEPS);
from 45 to 99.5% by weight, preferably from 51 to 99.5% by weight, of a plasticising substance or a mixture of plasticising substances, which are liquid at room temperature. As non-limiting examples the plasticiser can be water, various alcohols (like in particular glycerol), glycols and their ethers, polyglycols, liquid polybutenes, esters such phthalates, adipates, stearates, palmitates, sebacates, or myristates, natural or synthetic oils such as vegetable oils, mineral oils, or combinations thereof;
from 0% to 50% by weight of the composition, preferably from 0 to 600% by weight of the macromolecular polymeric substance of a tackifying resin whose main scope is to tailor the Tg especially in systems based on synthetic polymers;
from 0 to 10% and more preferably form 0 to 5% by weight of substances for facilitating and stabilising the gel and the gel forming process both of hydrophilic or hydrophobic liquid plasticisers. These may be for oily systems, e.g. the fatty acids of C₈ to C₂₂, their metallic salts and their polyoxo-derivatives; lanolin derivatives; silica; bentonite, montmorillonite and their derivatives; polyamides, waxes or mixtures thereof.

The adhesive may also be of the type described in US Patent No. 6,213,993 to Zacharias et al. In particular the adhesive may comprise:
a rubber-based adhesive such as styrenebutadiene, polyisobutylene, polybutadiene and polyisoprene; a water soluble adhesive such as polyvinyl alcohol, polyvinyl acetate, and methyl cellulose; a hot melt adhesive such as block copolymers of styrene-butadiene-styrene, styrene-isoprene-styrene, styrene-ethylenepropylene-styrene, styrene-ethylenebutylene-styrene and tetrablock copolymers such as styrene-ethylenepropylene-styrene-ethylenepropylene. Incorporated with the adhesives can be suitable tackifying resins and, if appropriate, oils.

Other adhesive types here include anhydrous gels consisting of 2-hydroxyethyl methacrylate polymer, polyethylene glycol and optionally water as taught in U.S. Patent No. 4,303,066 and polyurethane gels, as disclosed in U.S. Patent No. 4,661,099 , or silicone gels including commercial products such as Silgel 612 from Wacker Silicones (Adrian, MI) or SSA-9700 Soft Skin Adhesives Dow-Corning (Midland, MI).

The adhesive 33 is covered prior to use by a removable release member 47. As shown in Fig 4a, the release member 47 may comprise a laminate structure including a first layer 49 formed from a cellulosic paper and a film coating 51 applied to the internal surface of layer 49. The film coating 51 is preferably formed from either polypropylene or polyethylene. Polycoated papers of this type are commercially available, for example POLY SLIK® brand papers are available from Loparex Inc., Willowbrook, IL. The internal surface of layer 51 is provided with a release coating 53. The release coating 53 is arranged such that prior to the removal of the release member 47 the coating 53 is in abutting face to face relationship with the adhesive 33. The release coating 53 facilitates the removal of the release member 47 prior to use. The film coating 51 preferably has a thickness between about .4 mils and about 2 mils and more preferably between about .6 to about 1.2 mils.

As shown in Fig. 4b, the release member 47 may alternatively comprise a sheet 55 of nonporous film such as polypropylene or polyethylene. The internal surface of sheet 55 is provided with a release coating 53 to facilitate the removal of the release member 47 prior to use. The sheet 55 preferably has thickness between about .5 mils and about 2 mils and more preferably between about .6 and about 1.2 mils.

Reference is made to the CD-Rom "PDL Chemical Resistance of Plastics and Elastomers" published William Andrew Publishing, © 2001, ISBN 1-884207-90-1. Reference is made to the "Mineral Oil Exposure Medium Table" provided in the above referenced publication, said table discloses PDL ratings of various materials. The PDL rating is a measure of the chemical resistively of the particular listed material to an exposure medium. The film coating 51 and/or the film sheet 55 is preferably made from a material having a PDL rating of at least 7, preferably at least 8, and most preferably at least 9 wherein the exposure medium is mineral oil at 20° C.

The release coating 49 may be a material based on polydimethylsiloxane chemistries, generically referred to as "silicones". Polydimethylsiloxane coatings are typically cured or crosslinked through a variety of methods. These methods may include crosslinked systems based on:
- Condensation reactions, such as those using tin catalysts, silanol functional siloxane, and silane hydride functional crosslinkers.
- Noble metal catylized systems using a vinyl-functional siloxane and a sliane-hydride functional polymer.
- Electron beam or ultraviolet light cured silicone chemistries which are based on epoxidized or acrylated siloxane moieties.
- Fluorosilicone chemistries based on acrylated fluorosiloxanes.

The release coating 49 may also be a material based on other non-silicone chemistries, such as fluropolymers, alkyds, carbamates, urethanes, chromium complexes, acrylics, poly vinyl alcohols, or olefins.

The release member 47 as described above prevents the migration of the adhesive 33 through the release member. Thus a stain does not form on the surface of the release member, resulting in a aesthetic pleasing and hygienic appearance, nor is the body attachment performance of the adhesive compromised.

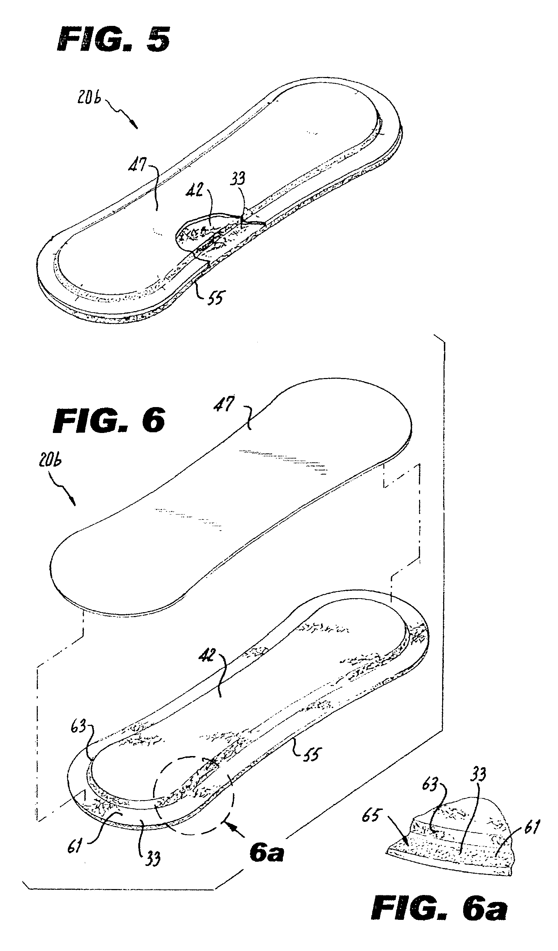

Another embodiment of the present invention, a sanitary napkin 20b, is shown in Figs. 5 and 6. In the sanitary napkin 20b the barrier layer 55 is dimensioned so a portion 61 thereof extends outward relative to an terminal edge 63 of the cover 42. The adhesive 33 for attaching the article to the body is applied to a body facing surface 65 of the barrier portion 61. The napkin 20b is provided with a removable release member 47 to cover the adhesive 33 prior to use. As shown in Figs. 5 and 6,the release member 47 may be shaped such that it extends over the entire top surface of the cover 42 and barrier portion 61. Alternatively, the removable release member 47 may have a substantially oval shape (not shown) such that the release member 47 corresponds in shape to the barrier portion 61 and has a central open area (i.e. a central oval shaped through hole) that corresponds to the shape of the cover 42. In all other respects the embodiment of the invention shown in Figs. 5 and 6 is the same as the embodiment described above with reference to Figs. 2- 4b.

### Main Body--Cover Layer

The cover layer 42 may be a relatively low density, bulky, high-loft non-woven web material. The cover layer 42 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 42 has a basis weight in the range of about 10 gsm to about 75 gsm.

Bi-component fibers may be made up of a polyester layer and a an polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 42 should not lose their physical properties when they are wetted, in other words they should not collapse or lose their resiliency when subjected to water or body fluid. The cover layer 42 may be treated to allow fluid to pass through it readily. The cover layer 42 also functions to transfer the fluid quickly to the other layers of the absorbent system 44. Thus, the cover layer 42 is advantageously wettable, hydrophilic and porous. When composed of synthetic hydrophobic fibers such as polyester or bi-component fibers, the cover layer 42 may be treated with a surfactant to impart the desired degree of wettability.

In one preferred embodiment of the present invention the cover is made from a spunlace nonwoven material having from about 0 to about 100% polyester and from about 0 to about 100% rayon. The spunlace material may also be made from about 10% to about 65% rayon and from about 35% to about 90% polyester. In lieu of, and/or in combination with the polyester, polyethylene, polypropylene or cellulosic fiber may be used with the rayon. Optionally, the material used for the cover layer may include binders such as thermoplastic binders and latex binders.

Alternatively, the cover layer 42 can also be made of polymer film having large pores. Because of such high porosity, the film accomplishes the function of quickly transferring body fluid to the inner layers of the absorbent system. Apertured coextruded films such described in U.S. Pat. No. 4,690,679 and available on sanitary napkins sold by Johnson & Johnson Inc. of Montreal, Canada could be useful as cover layers in the present invention.

The cover layer 42 may be embossed to the remainder of the absorbent system 44 in order to aid in promoting hydrophilicity by fusing the cover to the next layer. Such fusion may be effected locally, at a plurality of sites or over the entire contact surface of cover layer 42 and absorbent system 44. Alternatively, the cover layer 42 may be attached to the absorbent system 44 by other means such as by adhesion.

### Main Body -- Absorbent System

The absorbent system 44 may comprise a single layer of material or may comprise multiple layers. In one embodiment, the absorbent system 44 is a blend or mixture of cellulosic fibers and superabsorbent disposed in and amongst fibers of that pulp.

Cellulosic fibers that can be used in the absorbent system 44 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The absorbent system 44 can contain any superabsorbent polymer (SAP), which SAPs are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.

### Main Body -- Barrier Layer

Underlying the absorbent layer 44 is a barrier layer 50 comprising liquid-impervious film material so as to prevent liquid that is entrapped in the absorbent system 44 from egressing the sanitary napkin and staining the wearer's undergarment. The barrier layer 50 is preferably made of polymeric film, although it may be made of liquid impervious, air-permeable material such as repellent-treated non-woven or micropore films or foams.

The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 42 and the barrier layer 50 are joined along their marginal portions so as to form an enclosure or flange seal that maintains the absorbent layer 44 captive.

Any or all of the cover, absorbent layer, transfer layer, backsheet layer, and adhesive layers may be colored. Such coloring includes, but is not limited to, white, black, red, yellow, blue, orange, green, violet, and mixtures thereof. Color may be imparted according to the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The absorbent article may include other known materials, layers, and additives, such as, foam, net-like material, perfumes, medicaments or pharmaceutical agents, moisturizers, odor control agents, and the like. The absorbent article can optionally be embossed with decorative designs.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated by the present invention are asymmetrical and symmetrical absorbent articles having parallel longitudinal edges, dog bone- or peanut-shaped, as well as articles having a tapered construction for use with thong-style undergarments.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practising the present invention.

## Claims

1. An absorbent article comprising:
a liquid pervious cover;
a liquid impermeable barrier;
a body facing surface;
an adhesive for securing the article to the body arranged on the body facing surface; and
a removable release member for covering the adhesive prior to use, said release member being impervious to the migration of any component of said adhesive therethrough.

2. The absorbent article according to claim 1, wherein said removable release member is a laminate structure comprising a first layer formed from cellulosic paper and at least a second layer formed from a nonporous film coating.

3. The absorbent article according to claim 2, wherein said release member comprises a coating of silicone.

4. The absorbent article according to claim 2, wherein said film coating is polypropylene or polyethylene.

5. The absorbent article according to claim 1, wherein said removable release member is formed from a nonporous film material.

6. The absorbent article according to claim 5, wherein said film material is polypropylene or polyethylene.

7. The absorbent article according to claim 5 or claim 6, wherein said release member further comprises a coating of silicone.

8. The absorbent article according to any one of claims 2 to 4 or any one of claims 5 to 7, wherein said film coating or film material, respectively, has a PDL rating of at least 7, preferably at least 8, more preferably at least 9, when an exposure medium is mineral oil at a temperature of 20° C.

9. The absorbent article according to any one of claims 1 to 8, wherein said adhesive has a liquid plasticizer content of more about 50% by weight, preferably more than about 65% by weight, more preferably more than about 80% by weight.

10. The absorbent article according to claim 9, wherein said liquid plasticizer has a molecular weight of less than 1000 g/mole, preferably less than 750 g/mole, more preferably less than 500 g/mole.

11. The absorbent article according to any one of claims 1 to 10, wherein said adhesive is an adhesive having a linear or radial co-polymer structure having the formula (A-B)ₓ wherein block A is a polyvinylarene block, block B is a poly(monoalkenyl) block, x denotes the number of polymeric arms, and wherein x is an integer greater than or equal to one.

12. The absorbent article according to claim 11, wherein said block A polyvinylarene is Polystyrene, Polyalpha-methylstyrene, Polyvinyltoluene or a combination thereof.

13. The absorbent article according to claim 12, wherein said block B poly(monoalkenyl) is a conjugated diene elastomer including polybutadiene, polyisoprene, a hydrogenated elastomer including ethylene-butylene or ethylene-propylene or polyisobutylene or a combination thereof, an adhesive consisting of styrene-ethylene-butylene-styrene (SEBS) block copolymer and a mineral oil, a paraffinic or napthenic process oil, and optionally a suitable tackifying resin including a natural or modified resin; a glycerol or pentaerythritol ester of a natural or modified resin; a polyterpene resin; a copolymers or terpolymer of a natural terpene; a phenolic modified terpene resin or a hydrogenated derivative thereof; an aliphatic petroleum resin or a hydrogenated derivative thereof; an aromatic petroleum resin or a hydrogenated derivative thereof; or an aliphatic/aromatic.

14. The absorbent article according to any one of claims 1 to 13, wherein said cover is made from a spunlace nonwoven material having from 0 to 100% polyester and from 0 to 100% rayon, prefereably from 10% to 65% rayon and from 35% to 90% polyester.

15. The absorbent article according to any one of claims 1 to 13, wherein said cover is made from an apertured film material.

16. The absorbent article according to any one of claims 1 to 15, wherein said barrier is formed from a breathable film material.

17. The absorbent article according to any one of claims 1 to 16, which is a sanitary napkin, a liner or an adult incontinence product.

18. An absorbent article comprising:
a liquid pervious cover;
a liquid impermeable barrier;
a body facing surface;
an adhesive for securing the article to the body arranged on the body facing surface, wherein said adhesive is an adhesive gel based upon a styrene-ethylene-butylene-styrene (SEBS) block copolymer;
a removable release member for covering the adhesive prior to use, said release member being impervious to the migration of any component of said adhesive therethrough.

19. A method for preventing a migration of an adhesive though a removable release member in a body attachable sanitary article, comprising:
providing a body facing surface;
applying an adhesive to the body facing surface for securing the article to a user's body;
applying a removable release member over said adhesive to protect the adhesive prior to use;
providing said release member with a nonporous structure to prevent the migration of any component of said adhesive through said release member.
